(19) Europäisches Patentamt

European Patent Office

Office européen des brevets

(11) **EP 1 187 640 B1**

(12) **EUROPEAN PATENT SPECIFICATION**

(45) Date of publication and mention
of the grant of the patent:
**27.09.2006 Bulletin 2006/39**

(51) Int Cl.:
***A61L 15/00*** (2006.01)

(21) Application number: **00925720.5**

(22) Date of filing: **08.05.2000**

(86) International application number:
**PCT/KR2000/000430**

(87) International publication number:
**WO 2000/071176 (30.11.2000 Gazette 2000/48)**

(54) **WATER-ABSORBENT RESIN COMPOUND AND PROCESSING**

WASSERABSORBIERENDES HARZ UND VERFAHREN

COMPOSE A BASE DE RESINE HYDROPHILE ET PROCEDE

(84) Designated Contracting States:
**BE DE DK ES FR GB IT**

(30) Priority: **24.05.1999 KR 9918637**

(43) Date of publication of application:
**20.03.2002 Bulletin 2002/12**

(73) Proprietor: **Kolon Chemical Co., Ltd.**
**Kwacheon-city,**
**Kyunggi-do 427-040 (KR)**

(72) Inventors:
• **CHUN, Bo, Kyung**
**Anyang-city,**
**Kyunggi-do 431-060 (KR)**
• **KANG, Bong, Seo**
**Inchon-city 404-753 (KR)**
• **KANG, Myung, Koo**
**Buchon-city,**
**Kyunggi-do 420-110 (KR)**

(74) Representative: **Merkle, Gebhard**
**TER MEER STEINMEISTER & PARTNER GbR,**
**Patentanwälte,**
**Mauerkircherstrasse 45**
**81679 München (DE)**

(56) References cited:
**EP-A1- 0 457 660**          **EP-A2- 0 668 080**
**US-A- 4 587 308**          **US-E- R E33 839**

EP 1 187 640 B1

**Description**

TECHNICAL FIELD

This invention relates to a water-absorbent resin compound and a method for the production.

More particularly, it relates to a water-absorbent resin compound and a method for the production which possesses an excellent absorption capacity for urine, secretion and other body liquids and removes such malodors as ammonia and triethylamine resulting from the decomposition of urine and secretion even after long wearing of sanitary materials and prevents from skin hives.

DESCRIPTION OF THE RELATED ART

Heretofore, many water-absorbent resins have been commercialized such as the partially neutralized and cross-linked poly-acrylic acids, hydrolyzed starch-acrylonitrile graft polymers, neutralized starch-acrylic acid graft polymers, neutralized vinyl acetate-acrylic acid ester copolymers, hydrolyzed acrylonitrile copolymers or hydrolyzed acrylamide copolymers. They are produced by aqueous solution polymerization, suspension polymerization and reversed-phase suspension polymerization.

Above mentioned water-absorbent resins can absorb the aqueous liquids and they are mainly used for sanitary materials as well as for agricultural materials and soil melioraters.

The water-absorbent resin of this class have heretofore been desired to possess good properties like high water-absorption capacity and fine speed of absorption on contact with aqueous liquids, liquid permeability and high strength of the gel swelled with liquid.

Recently, however, the sanitary materials like disposable diapers and sanitary napkins are needed to possess not only the power of absorbing the body liquids like urine and secretions but the malodorproofing effect even after wearing sanitary materials for a long time and the preventive function from erythema and irritation on the skin on the contact with urine.

If people wear the sanitary napkins made of the former water-absorbent resins for a long time, particular malodors like amines raised by effluvia resulting from the decomposition of urine and secretions on the sanitary materials by microbes. Especially, the ammonia which is so volatile, contacts human skin and becomes one of the reasons for dermatitis.

So, many methods were proposed to treat the materials with additives and chemicals on the top sheet nonwoven fabric which constructs the sanitary materials to solve the former problems above mentioned. These methods, however, have not been commercialized due to its unfavorable affects onto body on the contact of the chemicals of the top sheet nonwoven fabric with the skin.

Therefore, many methods of treatment with additive and chemicals into the water-absorbent resins and fiber of top sheet nonwoven fabric have been tried in order to solve the problem of skin hives and malodors without side-effects to body.

More particularly, the method of adding antibiotics with nitrogen into the water-absorvent resin or the fiber of top sheet nonwoven fabric have been disclosed in US patent 4.842,593 and Japanese patent publication 1998-168757.

The method of adding the metals like aluminum, copper or siliver into the water-absorbent resin or the fiber of top sheet nonwoven fabric have been disclosed in Japanese patent publication 1993-212094 and Japanese patent publication 1995-165981 disclosed.

The method of adding the multicavernous inorganic materials like zeolite into the water-absorbent resin or the fiber of top sheet nonwoven fabric have been disclosed in Japanese patent publication 1993-161671 and Japanese patent publication 1996-176338.

But the methods adding antibiotics with nitrogen into the water-absorbent resins and the fiber of top sheet nonwoven fabric can not be commercialized because they are easy to influence side-effects onto the body, in spite of the positive effects to kill the microbes making malodors. The methods adding multicavernous inorganic materials like zeolite into the water-absorbent resins and the fiber of top sheet nonwoven fabric have less side-effects onto the body and better malodorproofing function, especially in gas. But it can not get rid of the malodors efficiently if the sanitary material has been weared for a long time, due to its less capacity of defogging the malodors in liquids.

More illustration follows on the malodorproofing effect of zeolite below. The secretion and urine come out of the body i n liquids and they get slowly decomposed by the microbes and produce malodors from the residue. The malodors in gas hide in liquids and emit slowly by the passage of time and it is different from the status where they move merely as gas in the air.

Once the microcavities of zeolites are filled with the liquids of secretions and urines, the zeolites can not have the malodorproofing effect any more.

The purpose of the present invention provides a capable water-absorbent resins, and a process for preparing the same that has excellent absorption capacity, malodorproofing effect and the preventing function from skin hives by eliminating the drawbacks of former methods described hereinabove.

SUMMARY OF THE INVENTION

The present invention relates to a water-absorbent resin compound and methods for the production. More particularly, it relates to a water-absorbent resin compound comprising 0.1-30 weight part of an monomer containing the carboxyl radical and at most 10 weight parts of filler of fumed silica to 100 weight part of a water-absorbent resin.

The present invention also relates to the production method of adding properly or spraying the solution of the monomer containing the carboxyl radical over the processing or the produced water-absorbent resins and selectively add the filler of fumed silica.

Recently, one of the most common water-absorbent resins comprising the structure of the carboxyl radical(-COOH) and the sodium salt(-COONa) is partly neutralized and cross-linked poly-acrylic acid sodium salt. The said carboxyl radical and the said amines are expected to have the malodorproofing effect with mechanism in accordance with the following formula (I).

$$\text{Water-absorbent resin -COOH+NR'3} \rightarrow \text{R-COONHR'3(Malodorproof)} \qquad \text{(I)}$$

The NR'3 in the above formula is ammonia or triethylamine.

Considering the above mechanism, the inventor noticed if he increases the carboxyl radical in the water-absorbent resin, he can remove the old problems such as malodors from the amines caused by the adoption of antibiotics, metals and zeolites into the sanitary materials and prevent the skin hives as well.

Therefore, the present invention has the characteristic quality that it sustains the basic property of the water-absorbent resins but increases its malodorproofing capacity and makes it prevent the skin hives, adding the monomers with high amount of the carboxyl radical into the water-absorbent resins.

The water-absorbent resin compound in the present invention can be used for various kinds of body liquids absorption goods such as the sanitary napkins, and disposable diapers and be applicable for the injury protecting materials and curing instruments for the incontinent.

BRIEF DESCRIPTION OF THE DRAWING

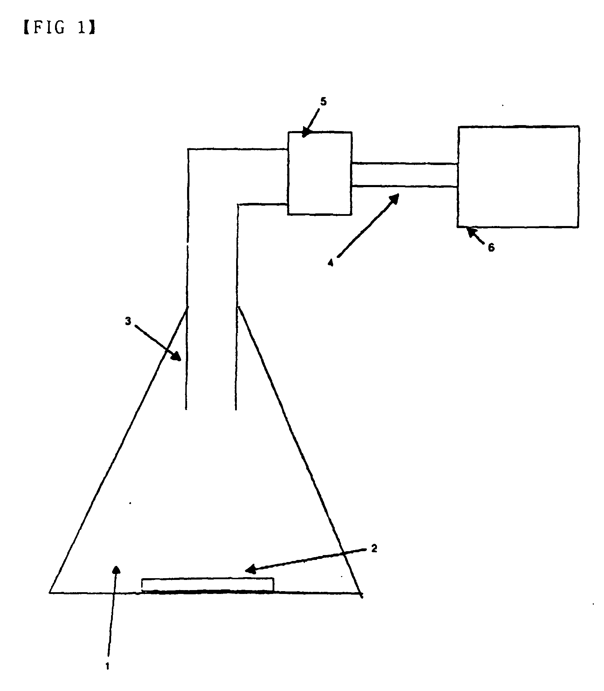

FIGURE1 is the overview about the device to measure with the malodorproofing effect of the water-absorbent resin in the invention.

In FIGURE1, 1 refers to a flask, 2 to the tested material. 3 to a vacuum adaptor. 4 to a measuring pipe, 5 to a silicon connecting tube, and 6 to a gas collector.

DETAILED DESCRIPTION OF PREFERRED EMBODIMENTS

The resin compound in this invention comprises the water-absorbent resin, the monomers containing the carboxyl radical and the filler of fumed silica.

The monomers containing the carboxyl radical serves to increase the malodorproofing effect, maximizing the ratio of the carboxyl radical in the water-absorbent resin, and to prevent the skin hives. The filler of fumed silica works to enhance the flowability of the water-absorbent resin.

The water-absorbent resin used in the present invention includes partially neutralized and cross-linked poly-acrylic acids, hydrolyzed starch-acrylonitrile graft polymers, neutralized starch-acrylic acid graft polymers, neutralized vinyl acetate-acrylic acid ester copolymers, hydrolyzed acrylonitrile copolymers or hydrolyzed acrylamide copolymers.

In this invention, the monomers containing carboxyl radical is one or more kinds of compound selected from i) food additives like acetic acid, lactic acid, citric acid, tartaric acid and succinic acid, ii) interface activators like lauric acid, myristic acid, palmitic acid, stearic acid, behenic acid, oleic acid, linolenic acid, arachidic acid andricinoleic acid, iii) organic acids like vitamins, and iv) other acids like amino acid, benzoic acid, pyruric acid, fumaric acid, maleic acid, propionic acid, bytyric acid, caproic acid, oxalic acid, aliginic acid, ethoxyacetic acid, glucuronic acid, sal icyl ic acid, cinamic acid, deoxycholic acid and allofuranoic acid.

From the viewpoint of body safety, it is recommended to use the food additives like acetic acid, lactic acid, citric acid, tartaric acid and succinic acid or the organic acids like vitamins as monomers containing the carboxyl radical.

But this invention does not specifically limit the classes of monomers containing the carboxyl radical.

The amount of monomers containing the carboyl radical in the water-absorbent resin compounds of the present invention is from 0.1 to 30 parts by weight, more preferably from 0.2 to 15 parts by weight or most preferably from 0.5 to 10 parts by weight to 100 parts by weight of a water-absorbent resin. If more than 30 parts by weight are used, it is not economic and the flowability of the water-absorbent resin slows down so rapidly that the production effectivity of sanitary napkins and the absorption capacity of urine and secretion decreases.

In the mean time, the amount of the filler of fumed silica is less than 10 parts by weight or more preferably less than 5 parts by weight, most preferably less than 3 parts by weight to 100 parts by weight of a water-absorbent resin. I the amount of the filler is over 10 parts by weight, the water-absorbent resin tend to stick together, so that they show decreased material 1 properties of water absorbent resin such as the absorption capacity under load.

The following description relates to the process for preparing the water-absorbent resins of the present invention.

First of all, dissolve the selected monomer containing the carboxyl radical into a solvent, and prepare the solution composed thereof. At the moment, water or water and hydrophlic organic solvents are used as the solvent. In the production of the monomer solution containing the carboxyl radical, the amount of the solvent depends on the kinds of monomer containing the carboxyl radical and the water-absorbent resins but most of the time it is from 1 to 30 parts by weight and more preferably from 3 to 20 parts by weight to 100 parts by weight of a water-absorbent resin. If the solvent is less than 1 part by weight, it is difficult to equally treat the monomers containing the carboxyl radical. If the solvent is more than 30 parts by weight, it takes longer to dry and decreases the effective and economic productivity.

The above mentioned monomer solution is sprayed over or properly mixed into the processing or produced water-absorbent resins. In this process, rotating mixer, screwing mixer, and level floating dryer can be used for the purpose of equal mixing.

To spray and coat the monomer containing the carboxyl radical directly over the produced water-absorbent resins is most desirable because it can maximize the malodorproofing effect and retain the original material property of the resins.

After the above mentioned mixing process, heat treatment can be made to help the safe adherence of the monomer containing the carboxyl radical. In the heat treatment, the temperatures are from 30 to 200°C, more preferably from 50 to 150°C and most preferably from 60 to 100°C.

In the present invention, the monomer solution containing the carboxyl radical is directly sprayed over or mixed into the water-absorbent resins and the filler which is hydrophilic or Fumed Silica is mixed thereto. The equipments such as the batch type Tumbler mixer and Henkel mixer can be used at this process.

From 0.1 to 30 parts by weight of the monomer containing the carboxyl radical and from at most 10 parts by weight of the filler of fumed silica are mixed to 100 parts by weight of the water-absorbent resin.

Among the water-absorbent resin compounds which were produced in accordance with the above mentioned methods in the present invention, the monomer containing the carboxyl radical increase content of carboxyl radical in the resin and helps it have the malodorproofing effect and prevent from skin hives. The filler of fumed silica serves to increase the flowability of the water-absorbent resin and prevent the resins from sticking together.

As a result, the water-absorbent resin compound in the present invention is excellent in having the malodorproofing effect and preventing from the skin hives, maintaining its original material property.

The water-absorbent resin compounds in the present invention has the absorption capacity without load of at least 30g/g to saline and the suction power of at least 5g/g to the pig's blood.

In the present invention, the method to measure the property of the water-absorbent resin and its malodorproofing effect is as follows.

- Measurement of the absorption capacity to physiological saline

After 0.5g of water-absorbent resin has been left in the 100g of 0.9% saline for one hour, it is taken out with the ASTM 100 mesh standard net. Its absorption capacity(g/g) is measured in accordance with the amount of the absorbed saline.

- Measurement of the absorption capacity to pig's blood

After 1.0g of water-absorbent resin has been left in the 60g of fresh pig's blood for one hour, it is taken out with the ASTM 100 mesh standard net. Its absorption capacity (g/g) is measured in accordance with the amount of the absorbed blood.

- Measurement of the absorption speed to physiological saline

The absorption speed is measured in accordance with the lapse of the disappearing vortex when 2.0g of water-absorbent resin is added into the 50g of physiological saline which is revolving at 500rpm.

- Measurement of flowability

Check the time lapse of 100g of water-absorbent resin to pass the flowmeter funnel(JIS K-3362).

- Measurement of malodorproofing effect

In the present invention, a measuring equipment is used as in Figure 1 to measure the malodorproofing effect for the malodors emitted from the urine and secret ion in liquids as time passes.

More particularly, the test material(2) is put into the flask(1) which is a water-absorbent resin or the sanitary material made of it.
Then, the solution of odor solvent (ammonia or trimethylamine) solved in physiological saline or pig's blood which are similar to urine and secretion, is added into the flask(1) with pipet and the flask(1) is sealed up and left in an oven at 40°C for 4 hours.

Later, the flask (1) is opened and measure the amount (ppm) of existing odors in the air of flask using measuring pipe(-1) and gas collector (5). We refer the measured amount to A.

Meanwhile, without the test material (2) of water-absorbent resin or the sanitary napkins made of it, just the solution of odor solvent (ammonia or trimethylamine) solved, in physiological saline or pig's blood which are similar to urine and secretion, is added into the flask (1) and it is sealed up and left in an oven at 40°C for 4 hours.

Later, the flask (1) is opened and measure the amount (ppm) of existing odors in the air of flask using measuring pipe(4) and gas collector (5). We refer the measured amount to B.

The malodorproofing effect is calculated in accordance with the following formula.

$$\text{Malodorproofing effect}(\%) = \frac{(B-A)}{B} \times 100$$

The present invention will be more particularly recognized with the following examples and comparative examples. But this invention is not confined in the following examples.

EXAMPLE 1

The solution is made in the way that 1 part by weight of amino acid and 2 parts by weight of cinamic acid are solved into the 5 parts by weight of methanol to 100 parts by weight of the acrylate sodium salt water-absorbent resin (Kolon Chemical Co. Ltd. K-SAM GS-3300N).

The produced solution is sprayed and coated over the surface of the above mentioned water-absorbent resin, dried in an hot wind oven at 70°C and left at ordinary temperature. Then 5 parts by weight of the filler of fumed silica are mixed into it and the water-absorbent resin compound is produced.

The 75 weight parts of the above mentioned water-absorbent resin compound is mixed with the 25 weight parts of crushed pulp, sprayed over with distilled water and pressured on to become the water-absorbent structure (sanitary material) with the compression density of 0.17g/cc and basis weight of 0.47g/m$^2$

Table2 shows the malodorproofing effect of the above mentioned water-absorbent resin compound and the water-absorbent structure (sanitary material) made of it, evaluated with the conditions on Table1. The malodorproofing effect is measured in accordance with the pre-described measuring methods with the equipment on Figure1.

TABLE 1. Evaluation Conditions for the Malodorproofing effect

|  | | Test material | Urine or alternative of secretion | Odor emitting elements |
|---|---|---|---|---|
|  | Condition 1 | Water-absorbent resin compound (1g) | Physiological saline (40g) | Ammonia (20 micro liter) |
|  | Condition 2 | Water-absorbent resin compound (1g) | Physiological saline (40g) | Triethylamine (10 micro liter) |
|  | Condition 3 | Waler-absorbent resin compound (1g) | pig blood (10g) | Ammonia (20 micro liter) |

(continued)

|  | Test material | Urine or alternative of secretion | Odor emitting elements |
|---|---|---|---|
| Condition 4 | Water-absorbent resin compound (1g) | pig blood (10g) | Triethylamine (10 micro liter) |
| Condition 5 | Water-absorbent structure (sanitary material) | Physiological saline (100g) | Ammonia (50 micro liter) |
| Condition 6 | Water-absorbent structure (sanitary material) | pig blood (10g) | Triethylamine (10 micro liter) |

Table3 shows the evaluation result of the material property of the produced water-absorbent resin compound.

EXAMPLE 2

A solution is produced in the way that 2 weight parts of succinic acid is solved into 5 weight parts of methanol and 5 weight parts of ethanol to 100 weight parts of starch acrylate water-absorbent resin (Sanwet IM-1000).
The solution is sprayed and coated over th.e above mentioned water-absorbent resin, dried in the hot wind oven at 60°C and left at the ordinary temperature. Then, 0.2 weight parts of filler of fumed silica is mixed to produce the water-absorbent resin compound.
Meanwhile, 75 weight parts of the above mentioned water-absorbent resin compound is mixed with 25 weight parts of crushed pulp, sprayed over with the distilled water, and pressured on to obtain the water-absorbent structure (sanitary material).
Table2 shows the malodorproofing effect of the water-absorbent resin compound and the water-absorbent structure (sanitary material) made of it evaluated with the conditions of Table1 in Example1. Table3 shows the result of the evaluation for the material property of the water-absorbent resin compound.

EXAMPLE 3

The solution is made in the way that 1 part by weight of ascobic acid and 2 parts by weight of tartaric acid are solved into the 5 parts by weight of distilled water to 100 parts by weight of the acrylate sodium salt water-absorbent resin (Kolon Chemical Co. Ltd. K-SAM GS-3400).
The produced solution is properly added and mixed over the surface of the above mentioned water-absorbent resin, dried in an hot wind oven at 100C and left at ordinary temperature. Then. 1 parts by weight of the filler of fumed silica is mixed to produce the water-absorbent resin compound.
Meanwhile, the 75 weight parts of the above mentioned water-absorbent resin compound is mixed with the 25 weight parts of crushed pulp, sprayed over with distilled water and pressured on to produce the water-absorbent structure (sanitary material).
Table2 shows the malodorproofing effect of the water-absorbent resin compound and the water-absorbent structure (sanitary material) made of it evaluated with the conditions of Table1 in Example1. Table3 shows the result of the evaluation for the material propeerty of the water-absorbent resin compound.

EXAMPLE 4

The solution is made in the way that 2 part by weight of maleic acid and 2 parts by weight of fumaric acid are solved into the 8 parts by weight of methanol to 100 parts by weight of the acrylate sodium salt water-absorbent resin (SNF Florger Co. Ltd., Flosorb-550).
The produced solution is properly added and mixed over the surface of the above mentioned water-absorbent resin, dried in an hot wind oven at 60°C and left at ordinary temperature. Then. 0.5 parts by weight of the filler of fumed silica is mixed to produce the water-absorbent resin compound.
Meanwhile. 75 weight parts of the above mentioned water-absorbent resin compound is mixed with the 25 weight parts of crushed pulp, sprayed over with distilled water and pressured on to produce the water-absorbent structure (sanitary material).
Table2 shows the malodorproofing effect of the water-absorbent resin compound and the water-absorbent structure (sanitary material) made of it evaluated with the conditions of Table1 in Example1. Table3 shows the result of the evaluation for the material property of the water-absorbent resin compound.

COMPARATIVE EXAMPLE 1

75 weight parts of the acrylate sodium sal t water-absorbent resin compound (GS-300N) without the monomer containing the carboxyl radical and the filler of fumed silica is mixed with the 25 weight parts of crushed pulp, sprayed over with distilled water and pressured on to produce the water-absorbent structure (sanitary material).
Table2 shows the malodorproofing effect of the water-absorbent resin compound and the water-absorbent structure (sanitary material) made of it evaluated with the conditions of Table1 in Example1. Table3 shows the result of the evaluation for the material property of the water-absorbent resin compound.

COMPARATIVE EXAMPLE 2

75 weight parts of the acrylate sodium salt water-absorbent resin compound (IM-1000) without the monomer containing the carboxyl radical and the filler of fumed silica is mixed with the 25 weight parts of crushed pulp, sprayed over with distilled water and pressured on to produce the water-absorbent structure (sanitary material).
Table2 shows the malodorproofing effect of the water-absorbent resin compound and the water-absorbent structure (sanitary material) made of it evaluated with the conditions of Table1 in Example1. Table3 shows the result of the evaluation for the material property of the water-absorbent resin compound.

COMPARATIVE EXAMPLE 3

75 weight parts of the acrylate sodium salt water-absorbent resin compound (GS-3400) without the monomer containing the carboxyl radical and the filler of fumed silica is mixed with the 25 weight parts of crushed pulp, sprayed over with distilled water and pressured on to produce the water-absorbent structure (sanitary material).
Table2 shows the malodorproofing effect of the water-absorbent resin compound and the water-absorbent structure (sanitary material) made of it evaluated with the conditions of Table1 in Example1. Table3 shows the result of the evaluation for the material property of the water-absorbent resin compound.

COMPARATIVE EXAMPLE 4

75 weight parts of the acrylate sodium salt water-absorbent resin compound (Flosorb-550) without the monomer containing the carboxyl radical and the filler of fumed silica is mixed with the 25 weight parts of crushed pulp, sprayed over with distilled water and pressured on to produce the water-absorbent structure (sanitary material).
Table2 shows the malodorproofing effect of the water-absorbent resin compound and the water-absorbent structure (sanitary material) made of it evaluated with the conditions of Table1 in Example1. Table3 shows the result of the evaluation for the material property of the water-absorbent resin compound.

COMPARATIVE EXAMPLE 5

75 weight parts of the acrylate sodium salt water-absorbent resin compound (Chemdal Co., ASAP-2102) without the monomer containing the carboxyl radical and the filler of fumed silica is mixed with the 25 weight parts of crushed pulp, sprayed over with distilled water and pressured on to produce the water-absorbent structure (sanitary material).
Table2 shows the malodorproofing effect of the water-absorbent resin compound and the water-absorbent structure (sanitary material) made of it evaluated with the conditions of Table1 in Example1.
Table3 shows the result of the evaluation for the material property of the water-absorbent resin compound.

TABLE 2. Evaluation result of malodorproofing effect

| | | | | | | [%] |
|---|---|---|---|---|---|---|
| | Evaluation Condition 1 | Evaluation Condition2 | Evaluation Condition 3 | Evaluation Condition 4 | Evaluation Condition 5 | Evaluation Condition 6 |
| Example 1 | 85 | 77 | 87 | 75 | 67 | 69 |
| Example 2 | 79 | 63 | 91 | 66 | 74 | 72 |
| Example 3 | 87 | 74 | 93 | 96 | 77 | 69 |
| Example 4 | 90 | 95 | 92 | 88 | 65 | 61 |
| Example 5 | 87 | 88 | 84 | 83 | 80 | 84 |

(continued)

| | Evaluation Condition 1 | Evaluation Condition2 | Evaluation Condition 3 | Evaluation Condition 4 | Evaluation Condition 5 | Evaluation Condition 6 [%] |
|---|---|---|---|---|---|---|
| Comp. Example1 | 29 | 30 | 34 | 33 | 24 | 32 |
| Comp. Example2 | 9 | 8 | 16 | 36 | 26 | 20 |
| Comp. Example3 | 10 | 9 | 3 | 16 | 22 | 26 |
| Comp. Example4 | 18 | 20 | 22 | 28 | 30 | 18 |
| Comp. Example5 | 10 | 14 | 16 | 16 | 21 | 25 |

TABLE 3. Result of the property test of the water-absorbent resin compound

| | Absorption to physiological Saline (g/g) | Absorption to pig blood (g/g) | Absorption speed (sec) | Flowability (sec) |
|---|---|---|---|---|
| Example 1 | 75 | 13 | 25 | 15 |
| Example 2 | 63 | 9 | 57 | 12 |
| Example 3 | 65 | 10 | 60 | 13 |
| Example 4 | 65 | 10 | 65 | 12 |
| Example 5 | 67 | 11 | 48 | 13 |
| Comparative Example 1 | 68 | 11 | 40 | 13 |
| Comparative Example 2 | 58 | 7 | 70 | 12 |

**Claims**

1. A resin compound comprising

   (a) water-absorbent resin,
   (b) a monomer having a carboxyl radical and
   (c) a filler of fumed silica,

   wherein the amount of the monomer (b) is 0.1-30 weight parts and the amount of the filler (c) is up to 10 weight parts, relative to 100 weight parts of the water-absorbent resin.

2. The resin compound according to claim 1, wherein the water absorbent resin is partially neutralized and cross-linked poly-acrylic acids, hydrolyzed starch-acrylonitrile graft polymers, neutralized starch-acrylic acid graft polymers, neutralized vinyl acetate-acrylic acid ester copolymers, hydrolyzed acrylonitrile copolymers or hydrolyzed acrylamide copoylmers.

3. The resin compound according to claim 1, wherein the monomer containing the carboxyl radical is one or more kinds of acids selected from acetic acid, lauric acid, myristic acid, palmitic acid, stearic acid, behenic acid, oleic acid, linolenic acid, arachidic acid, ricinoleic acid, ascorbic acid, amino acid, benzoic acid, pyruric acid, fumaric acid, maleic acid, propionic acid, bytyric acid, caproic acid, oxalic acid, aliginic acid, ethoxyacetic acid, glucuronic acid, salicylic acid, cinamic acid, deoxycholic acid and allofuranoic acid.

4. The resin compound according to claim 1, wherein the absorption capacity of the resin compound to saline without load is at least 30 g/g and the suction capacity to pig blood is at least 5 g/g.

5. A process for preparing the resin compound as specified in any one of claims 1 to 4 comprising the following two stages:

Firstly, the monomer solution containing the carboxyl radical (b) is sprayed over or properly added into the processing or produced water-absorbent resin (a);
Secondly, the filler of fumed silica (c) is mixed into it.

6. The process according to claim 5, wherein the absorbent, resin is obtained by properly adding or spraying 0.1-30 weight parts of monomer containing the carboxyl radical to 100 weight parts of absorbent resin.

7. The process according to claim 5, wherein the absorbent resin is obtained by adding or mixing up to 10 weight parts of fumed silica to 100 weight parts of absorbent resin.

8. The process according to claim 5, wherein the absorbent resin is partially neutralized and cross-linked poly-acrylic acids, hydrolyzed starch-acrylonitrile graft polymers, neutralized starch-acrylic acid graft polymers, neutralized vinyl acetate-acrylic acid ester copolymers, hydrolyzed acrylonitrile copolymers or hydrolyzed acrylamide copolymers.

9. The process according to claim 5, wherein the monomer containing the carboxyl radical is one or more kinds of acids selected from acetic acid, lauric acid, myristic acid, palmitic acid, stearic acid, behenic acid, oleic acid, linolenic acid, arachidic acid, ricinoleic acid, ascorbic acid, amino acid, benzoic acid, pyruric acid, fumaric acid, maleic acid, propionic acid, bytyric acid, caproic acid, oxalic acid, aliginic acid, ethoxyacetic acid, glucuronic acid, salicylic acid, cinamic acid, deoxycholic acid and allofuranoic acid.

**Patentansprüche**

1. Harzmischung, umfassend

(a) wasserabsorbierendes Harz,
(b) ein Monomer mit einem Carboxylrest, und
(c) einen Füllstoff aus Kieselpuder,

wobei die Menge des Monomeren (b) 0,1-30 Gewichtsteile und die Menge des Füllstoffs (c) bis zu 10 Gewichtsteile beträgt, bezogen auf 100 Gewichtsteile des wasserabsorbierenden Harzes.

2. Harzmischung nach Anspruch 1, wobei das wasserabsorbierende Harz aus teilweise neutralisierten und vernetzten Polyacrylsäuren, hydrolysierten Stärke-Acrylnitril-Pfropfpolymeren, neutralisierten Stärke-Acrylsäure-Pfropfpolymeren, neutralisierten Vinylacetat-Acrylsäureester-Copolymeren, hydrolysierten Acrylnitrilcopolymeren oder hydrolysierten Acrylamidcopolymeren besteht.

3. Harzmischung nach Anspruch 1, wobei das Monomer, das einen Caboxylrest enthält, besteht aus einer oder mehreren Arten von Säuren, gewählt aus Essigsäure, Laurinsäure, Myristinsäure, Palmitinsäure, Stearinsäure, Behensäure, Oleinsäure, Linolensäure, Arachidinsäure, Rizinolsäure, Ascorbinsäure, Aminosäure, Benzoesäure, Pyrurinsäure, Fumarsäure, Maleinsäure, Propionsäure, Buttersäure, Capronsäure, Oxalsäure, Alginsäure, Ethoxyessigsäure, Glucuronsäure, Salicylsäure, Zimtsäure, Desoxycholsäure und Allofuransäure.

4. Harzmischung nach Anspruch 1, wobei die Absorptionskapazität der Harzmischung gegenüber Salzlösung ohne Last mindestens 30 g/g und die Saugkapazität gegenüber Schweineblut mindestens 5 g/g beträgt.

5. Verfahren zur Herstellung einer Harzmischung nach mindestens einem der Ansprüche 1 bis 4, umfassend die folgenden zwei Schritte:

Erstens, die Lösung des den Carboxylrest enthaltenden Monomeren (b) wird gesprüht über oder in geeigneter Weise zugesetzt zu dem in der Herstellung befindlichen oder erzeugten wasserabsorbierenden Harz (a); Zweitens, der Füllstoff aus Kieselpuder (c) wird darin eingemischt.

**6.** Verfahren nach Anspruch 5, wobei das absorbierende Harz erhalten wird durch geeignetes Hinzugeben oder Sprühen von 0,1-30 Gewichtsteilen des den Carboxylrest enthaltenden Monomeren zu 100 Gewichtsteilen des absorbierenden Harzes.

**7.** Verfahren nach Anspruch 5, wobei das absorbierende Harz erhalten wird durch Zugeben oder Vermischen von bis zu 10 Gewichtsteilen von Kieselpuder zu 100 Gewichtsteilen des absorbierenden Harzes.

**8.** Verfahren nach Anspruch 5, wobei das absorbierende Harz aus teilweise neutralisierten und vernetzten Polyacrylsäuren, hydrolysierten Stärke-Acrylnitril-Pfropfpolymeren, neutralisierten Stärke-Acrylsäure-Pfropfpolymeren, neutralisierten Vinylacetat-Acrylsäureester-Copolymeren, hydrolysierten Acrylnitrilcopolymeren oder hydrolysierten Acrylamidcopolymeren besteht.

**9.** Verfahren nach Anspruch 5, wobei das den Carboxylrest enthaltende Monomer besteht aus einer oder mehreren Arten von Säuren, gewählt aus Essigsäure, Laurinsäure, Myristinsäure, Palmitinsäure, Stearinsäure, Behensäure, Oleinsäure, Linolensäure, Arachidinsäure, Rizinolsäure, Ascorbinsäure, Aminosäure, Benzoesäure, Pyrurinsäure, Fumarsäure, Maleinsäure, Propionsäure, Buttersäure, Capronsäure, Oxalsäure, Alginsäure, Ethoxyessigsäure, Glucuronsäure, Salicylsäure, Zimtsäure, Desoxycholsäure und Allofuransäure.

**Revendications**

**1.** Composé à base de résine comprenant

(a) une résine absorbant l'eau,
(b) un monomère ayant un radical carboxylique et
(c) une charge de silice fumée,

dans lequel la quantité du monomère (b) est de 0,1 - 30 parties en poids et la quantité de la charge (c) s'élève jusqu'à 10 parties en poids, par rapport à 100 parties en poids de résine absorbant l'eau.

**2.** Composé à base de résine selon la revendication 1 dans lequel la résine absorbant l'eau est constituée par des acides polyacryliques partiellement neutralisés et réticulés, des polymères greffés d'amidon-acrylonitrile hydrolysés, des polymères greffés d'amidon-acide acrylique neutralisés, des copolymères d'ester d'acide acrylique et d'acétate de vinyle neutralisés, des copolymères d'acrylonitrile hydrolysés ou d'acrylamide hydrolysés.

**3.** Composé à base de résine selon la revendication 1, dans lequel le monomère contenant le radical carboxylique est un ou plusieurs types d'acides choisis parmi l'acide acétique, l'acide laurique, l'acide myristique, l'acide palmitique, l'acide stéarique, l'acide béhénique, l'acide oléique, l'acide linoléique, l'acide arachidique, l'acide ricinoléique, l'acide ascorbique, les acides amino, l'acide benzoïque, l'acide pyrurique, l'acide fumarique, l'acide maléique, l'acide propionique, l'acide bytyrique, l'acide caproique, l'acide oxalique, l'acide aliginique, l'acide éthoxyacétique, l'acide glucuronique, l'acide salicylique, l'acide cinnamique, l'acide déoxycholique et l'acide allofunaroique,

**4.** Composé à base de résine selon la revendication 1, **caractérisé en ce que** la capacité d'absorption de la résine par rapport à une solution saline sans charge et d'au moins 30g/g et la capacité de succion par rapport au sang de porc est d'au moins 5g/g.

**5.** Un procédé pour préparer le composé à base de résine tel que défini dans l'une des revendications 1 à 4 comprenant les deux étapes suivantes :

premièrement, la solution de monomère contenant le radical carboxylique (b) est dispersée sur ou ajoutée proprement à la résine absorbant l'eau (a) au cours du procédé ou telle que produite ;
deuxièmement, la charge de silice fumée (c) est mélangée avec celle-ci.

**6.** Le procédé selon la revendication 5, dans lequel la résine absorbante est obtenue en ajoutant proprement ou en dispersant 0,1 - 30 parties en poids de monomère contenant la radical carboxylique par rapport à 100 parties en poids de résine absorbante.

**7.** Le procédé selon la revendication 5, dans lequel la résine absorbante est obtenue en ajoutant ou en mélangeant jusqu'à 10 parties en poids de silice fumée à 100 parties en poids de résine absorbante.

**8.** Le procédé selon la revendication 5, dans lequel la résine absorbant l'eau est constituée par des acides polyacryliques partiellement neutralisés et réticulés, des polymères greffés d'amidon-acrylonitrile hydrolysés, des polymères greffés d'amidon-acide acrylique neutralisés, des copolymères d'ester d'acide acrylique et d'acétate de vinyle neutralisés, des copolymères d'acrylonitrile hydrolysés ou d'acrylamide hydrolysés.

**9.** Le procédé selon la revendication 5, dans lequel le monomère contenant le radical carboxylique est un ou plusieurs types d'acides choisis parmi l'acide acétique, l'acide laurique, l'acide myristique, l'acide palmitique, l'acide stéarique, l'acide béhénique, l'acide oléique, l'acide linoléique, l'acide arachidique, l'acide ricinoléique, l'acide ascorbique, les acides amino, l'acide benzoïque, l'acide pyrurique, l'acide fumarique, l'acide maléique, l'acide propionique, l'acide bytyrique, l'acide caproique, l'acide oxalique, l'acide aliginique, l'acide éthoxyacétique, l'acide glucuronique, l'acide salicylique, l'acide cinnamique, l'acide déoxycholique et l'acide allofunaroïque.

【FIG 1】